# EUROPEAN PATENT APPLICATION

(11) **EP 4 803 903 A1**
(43) Date of publication of application: **09.09.2026**
(21) Application number: 25162358.3
(22) Date of filing: 07.03.2025
(51) Int. Cl.: G01N 33/564

(54) **AN IMPROVED QUANTITATIVE IMMUNOASSAY FOR THE DETECTION OF AUTOANTIBODIES TO NEPHRIN**

(71) Applicant: EUROIMMUN Medizinische Labordiagnostika AG, 23560 Lübeck (DE)
(72) Inventor: Rhein, Sina, 23560 Lübeck (DE); Mindorf, Swantje, 23560 Lübeck (DE); Radzimski, Christiane, 23560 Lübeck (DE); Probst, Christian, 23560 Lübeck (DE); Komorowski, Lars, 23560 Lübeck (DE)

(57) **Abstract**

The present invention relates to a method comprising the step detecting in a sample comprising antibodies from a mammalian subject the presence of an antibody to the extracellular domain of a mammalian nephrin, wherein the antibody is detected using a bridge capture assay and to a kit comprising (a) a first polypeptide comprising an epitope from the extracellular domain of a mammalian nephrin and a second polypeptide comprising an epitope from the extracellular domain of a mammalian nephrin, wherein the first polypeptide is immobilized on a solid carrier or configured for immobilization on a solid carrier and the second polypeptide is labeled or configured for labeling with a detectable label, wherein the kit comprises a solid carrier if the first polypeptide is configured for immobilization on a solid carrier, and wherein the kit comprises a detectable label if the second polypeptide is configured for labeling with a detectable label, or (b) a first polypeptide comprising an epitope from the extracellular domain of a mammalian nephrin and a second polypeptide comprising an epitope from the extracellular domain of a mammalian nephrin, wherein the first polypeptide is labeled label or configured for labeling with a first detectable label and the second polypeptide is labeled or configured for labeling with a second detectable label, wherein the kit comprises a first detectable label if the first polypeptide is configured for labeling with a first detectable label, and wherein the kit comprises a second detectable label if the second polypeptide is configured for labeling with a second detectable label.

## Description

The present invention relates to a method comprising the step detecting in a sample comprising antibodies from a mammalian subject the presence of an antibody to the extracellular domain of a mammalian nephrin, wherein the antibody is detected using a bridge capture assay, and to a kit comprising (a) a first polypeptide comprising an epitope from the extracellular domain of a mammalian nephrin and a second polypeptide comprising an epitope from the extracellular domain of a mammalian nephrin, wherein (i) the first polypeptide is immobilized on a solid carrier or (ii) configured for immobilization on a solid carrier (iii) or the kit comprises a solid carrier configured for the immobilization of the first polypeptide and the second polypeptide is labeled or configured for labeling with a detectable label, wherein the kit comprises a solid carrier if the first polypeptide is configured for immobilization on a solid carrier, and wherein the kit comprises a detectable label if the second polypeptide is configured for labeling with a detectable label, or (b) a first polypeptide comprising an epitope from the extracellular domain of a mammalian nephrin and a second polypeptide comprising an epitope from the extracellular domain of a mammalian nephrin, wherein the first polypeptide is labeled or configured for labeling with a first detectable label and the second polypeptide is labeled or configured for labeling with a second detectable label, wherein the kit comprises a first detectable label if the first polypeptide is configured for labeling with a first detectable label, and wherein the kit comprises a second detectable label if the second polypeptide is configured for labeling with a second detectable label.

Nephrin is an essential structural component of the slit diaphragm as illustrated by genetic mutations in nephrin (NPHS1) that cause complete lack of nephrin cell surface localization, underlying Congenital Nephrotic Syndrome of the Finnish Type (CNF). In contrast to congenital nephrotic syndrome with an established genetic basis, the cause of non-congenital nephrotic syndrome in both children and adults remains largely unknown. There is strong evidence supporting immune dysregulation with a potential causative circulating factor, however its identity has remained elusive. Glucocorticoids are effective at inducing remission, but relapses, steroid dependence and intolerance are common, often requiring alternative immunosuppressive agents. In those patients with steroid dependent nephrotic syndrome who progress to end stage kidney disease and require kidney transplantation, the disease can promptly recur in the allograft, a devastating and difficult-to-treat complication.

The discovery that anti-CD20 B-cell targeted therapies are effective in children with frequently relapsing or steroid-dependent nephrotic syndrome and in adults suggested a potential autoantibody-mediated etiology. Antibodies targeting the essential slit diaphragm component nephrin have been shown to cause massive proteinuria when administered in animal models and when they arise as alloantibodies following kidney transplantation in children with CNF and complete nephrin deficiency. In both animal models and cultured podocytes, anti-nephrin antibodies cause a redistribution of nephrin that is identical to that observed in renal biopsies of patients with nephrotic syndrome. This redistribution of nephrin away from the slit diaphragm along with separation of intercellular junctions between adjacent podocytes has long been proposed as a logical concept to explain the proteinuria in these patients. However, the cause of this redistribution remains unknown.

Recently, circulating autoantibodies against the extracellular domain of nephrin, the essential component of the podocyte slit diaphragm, were found in a subset of patients with non-congenital , childhood and adult-onset minimal change disease. These nephrin antibodies were specifically present in minimal change disease kidney biopsies, forming distinct clusters together with nephrin. The detection of these antibodies in blood samples from patients is therefore a non-invasive method for the diagnosis of minimal change disease (US2023/0393147).

The diagnostic value of antibodies to nephrin has been corroborated in further studies. Hengel *et al.* found that 44% of adult patients with minimal change disease were positive and 9% of those with primary focal segmental glomerulosclerosis (FSGS). The samples of 52% of a cohort of children with idiopathic nephrotic syndrome were also positive. In contrast, autoantibodies to nephrin could not be found in samples from patients suffering from other autoimmune diseases including ANCA-associated glomerulonephritis, lupus nephritis or from healthy controls (Hengel, F. E. et al. (2024) Autoantibodies Targeting Nephrin in Podocytopathies, N. Engl. J. Med. 391(5), 422-433).

In addition, the discovery of autoantibodies as a causative agent related to nephrological autoimmune diseases such as minimal change disease has paved the way for novel therapeutic approaches. Bressendorf *et al.* reported the successful treatment of a patient suffering from primary FSGS using plasmapheresis. This led to the complete clinical and immunological remission as monitored by regular detection of autoantibodies to nephrin in blood samples from the patient (Bressendorff, I. et al. (2024) Antinephrin-Associated Primary Focal Segmental Glomerulosclerosis Successfully Treated With Plasmapheresis, Kidney Int. Rep. 9, 2829-2831)

Watts *et al.* demonstrated that the concentration of antibodies to nephrin reflects the response to treatment and so can be used to monitor the progression of the disease or the success of any treatment (Watts A. J. B. et al. (2022) Discovery of Autoantibodies Targeting Nephrin in Minimal Change Disease Supports a Novel Autoimmune Etiology. J. Am. Soc. Nephrol. 33(1), 238-252).

Considering the medical importance of autoantibodies to nephrin, illustrated by applications such as the diagnosis of nephrological diseases associated with their presence, evaluating the severity and/or monitoring the progression of such diseases and ascertaining the success of treatment, there is an urgent need for methods that may be used to detect the antibodies in routine laboratories, for example for the purpose of diagnosing patients or monitoring their response to treatment. Several authors have published methods that were used for research. Wang *et al.* set up an ELISA method based on a mixture of two peptides, one from the intracellular and one from the extracellular domain of nephrin, to detect antibodies in sera from patients suffering from CNF (Wang, S. X. et al. (2001) Recurrence of nephrotic syndrome after transplantation in CNF is due to autoantibodies to nephrin, Exp Nephrol. 9(5), 327-31).

Batal *et al.* disclose the detection of pre-transplant anti-nephrin antibodies as a predictor of recurrent diffuse podocytopathy in the kidney allograft using a conventional ELISA (Batal, I. et al. (2024) Pre-transplant anti-nephrin antibodies are specific predictors of recurrent diffuse podocytopathy in the kidney allograft, Kidney Int.106(4), 749-752).

Patrakka *et al.* and Wang *et al.* also disclose the detection of autoantibodies to nephrin based on a conventional ELISA assay (Patrakka, J. et al. (2002) Recurrence of nephrotic syndrome in kidney grafts of patients with congenital nephrotic syndrome of the Finnish type: role of nephrin. Transplantation. 73(3), 394-403 and Wang, S. X. et al. (2001) Recurrence of nephrotic syndrome after transplantation in CNF is due to autoantibodies to nephrin. Exp Nephrol. 9(5), 327-31).

Hengel *et al.* recently reported efforts to detect autoantibodies to nephrin as markers of minimal change disease or idiopathic nephrotic syndrome using methods such as ELISA and direct Western blotting, but were unsuccessful (Hengel, F. E. et al. (2024) Autoantibodies Targeting Nephrin in Podocytopathies, N. Engl. J. Med. 391(5), 422-433). They speculated that the affinity or abundance of these autoantibodies in samples were too low. Therefore, a multi-step procedure was developed that involved concentrating (but not detecting) autoantibodies in samples using immunoprecipitation, followed by western blot analysis, similar to an assay described by Watts *et al.* (Watts A. J. B. et al. (2022) Discovery of Autoantibodies Targeting Nephrin in Minimal Change Disease Supports a Novel Autoimmune Etiology. J. Am. Soc. Nephrol. 33(1), 238-252). Samples were incubated overnight by Hengel *et al..* In addition, they used a combination of immunoprecipitation and ELISA. However, they found that the results of their assays did not correlate with disease activities and conclude that the sensitivity of their detection methods may leave room for improvement.

In any event, their approach is certainly not suitable for use in a routine clinical laboratory. First of all, the number of technical steps, comprising various steps for the immunoprecipitation for the purpose of concentrating the antibody and the subsequent detection is such that the resources required are prohibitive, in particular at a time when it is difficult to recruit skilled technicians. Results obtained by Western blot are semi-quantitative at best, while an accurate quantitative assay would be desirable for many applications, in particular monitoring patients, where any trend regarding the progression of a disease should be recognized as early as possible, or for testing drug candidates and their effect on patients. Finally, the assay relies on an overnight incubation, while it is strongly preferred that routine laboratories deliver results within one working day following receipt of patient samples. Therefore, assay formats contemplating a shorter incubation and/or fewer steps are required.

Therefore, the problem underlying the present invention is to provide an immunoassay, more specifically methods and products, which may be used to detect autoantibodies to nephrin reliably in a quantitative manner, in particular determining the absolute or relative concentration of the antibody.

Another problem underlying the present invention is to provide an immunoassay with a reduced number of steps that nonetheless detects the autoantibodies with high reliability, preferably improved to comparable state of the art assays, particularly in terms of sensitivity and specificity.

Another problem underlying the present invention is to provide an immunoassay which can be carried out more rapidly, ideally within few hours such that a sample delivered to a lab in the morning can be processed and a result communicated before the end of the working day. Preferably, an assay is provided which can be run with limited resources, in particular in terms of energy and/or working hours of qualified staff.

The problems underlying the present invention are solved by the subject-matter of the attached independent and dependent claims.

In a first aspect, the problem underlying the invention is solved by a method comprising the step detecting in a sample comprising antibodies from a mammalian subject the presence of an antibody to the extracellular domain of a mammalian nephrin, wherein the antibody is detected using a bridge capture assay.

In a second aspect, the problem underlying the invention is solved by a kit comprising (a) a first polypeptide comprising an epitope from the extracellular domain of a mammalian nephrin and a second polypeptide comprising an epitope from the extracellular domain of a mammalian nephrin, wherein (i) the first polypeptide is immobilized on a solid carrier or (ii) configured for immobilization on a solid carrier (iii) or the kit comprises a solid carrier configured for the immobilization of the first polypeptide and the second polypeptide is labeled or configured for labeling with a detectable label, wherein the kit comprises a solid carrier if the first polypeptide is configured for immobilization on a solid carrier, and wherein the kit comprises a detectable label if the second polypeptide is configured for labeling with a detectable label, or a first polypeptide comprising an epitope from the extracellular domain of a mammalian nephrin and a second polypeptide comprising an epitope from the extracellular domain of a mammalian nephrin, wherein the first polypeptide is labeled or configured for labeling with a first detectable label and the second polypeptide is labeled or configured for labeling with a second detectable label, wherein the kit comprises a first detectable label if the first polypeptide is configured for labeling with a first detectable label, and wherein the kit comprises a second detectable label if the second polypeptide is configured for labeling with a second detectable label.

In a preferred embodiment, the kit comprises one or more, preferably all from the group comprising a washing solution, a chemical solution reactive with the detectable label, a water-tight vessel, a positive control which is preferably an antibody to the extracellular domain of a mammalian nephrin, a calibrator, preferably a set of calibrators, a negative control, a blocking solution and a sample dilution buffer.

In a preferred embodiment, the solid carrier is selected from the group consisting of a glass slide, preferably for microscopy, a biochip, a microtiter plate, a lateral flow device, a test strip, a membrane, preferably a line blot, a chromatography column and a bead, preferably a magnetic or fluorescent bead.

In a preferred embodiment, the extracellular domain of a mammalian nephrin is from a mammalian nephrin (SEQ ID NO: 1), preferably selected from the group comprising human nephrin (SEQ ID NO: 2), murine nephrin (SEQ ID NO: 3), rat nephrin (SEQ ID NO: 4), rabbit nephrin (SEQ ID NO: 5), dog nephrin (SEQ ID NO: 6), cat nephrin (SEQ ID NO: 7), horse nephrin (SEQ ID NO: 8), sheep nephrin (SEQ ID NO: 9), goat nephrin (SEQ ID NO: 10) and camel nephrin (SEQ ID NO: 11), more preferably human nephrin (SEQ ID NO: 2).

In a preferred embodiment, the presence of the antibody is detected using a detection method selected from the group consisting of immunodiffusion, light scattering immunoassays, labeled immunoassays such as those from the group comprising radiolabeled immunoassay, enzyme immunoassays, chemiluminescence immunoassays, preferably electrochemiluminescence immunoassays, and fluorescence immunoassays.

In a preferred embodiment, the method or kit is for aiding in diagnosing whether a subject suffers from a nephrological autoimmune disease associated with the presence of antibodies to nephrin or for determining whether a sample comprising antibodies is from a subject more likely to suffer from a nephrological autoimmune disease associated with the presence of antibodies to nephrin than a healthy reference subject.

In a preferred embodiment, the method or kit is for monitoring the progression in a subject of a nephrological autoimmune disease associated with the presence of antibodies to nephrin. In a preferred embodiment, the method or kit is for evaluating in a subject suffering from a nephrological autoimmune disease associated with the presence of antibodies to nephrin the response to an immunosuppressive treatment of said disease.

In a preferred embodiment, the autoimmune disease is selected from the group comprising minimal change disease, FSGS and idiopathic nephrotic syndrome.

In a preferred embodiment, the method or kit is for evaluating the chances of a successful kidney transplantation or monitoring a subject or patient after a kidney transplantation.

In a preferred embodiment, the method or the kit is for detecting the binding of a drug or candidate drug to nephrin or for monitoring the efficacy of such a drug or candidate drug following administration to a patient.

In a preferred embodiment, the method or the kit is for selecting patients suffering from an autoimmune disease, preferably a nephrological autoimmune disease to be included in a cohort for a clinical study aiming to test the efficacy of immunosuppressive drugs, preferably systemic immunomodulators.

In a preferred embodiment, the subject is selected from the group comprising a primate, preferably a human, a mouse, a rat, a dog, a rabbit, a non-human primate, a guinea pig, a sheep, a goat, a camel, a horse, a hamster, an alpaca, a llama and a cow.

In a preferred embodiment, the method is or the kit is configured for the detection of the antibody in a sample which is selected from the group comprising whole blood, capillary blood, serum, plasma, and a dried blood spot, which is preferably moderately diluted only. In a preferred embodiment, the term "moderately diluted", as used herein, means that the sample if provided in the form of a bodily fluid, more preferably serum or plasma, is present, when in the presence of any of the first or the second polypeptide comprising mammalian, preferably human nephrin or an epitope thereof, at a dilution of no more than 1:10 (10% sample), 1:5 (20% sample), 1:3, 1:2 or 1:1.

In a preferred embodiment, the detection of the method is a qualitative detection, preferably a semi-quantitative detection, more preferably a quantitative detection or the kit is configured for a qualitative detection, preferably a semi-quantitative detection, more preferably a quantitative detection of the antibody.

The present invention is based on the surprising finding by the inventors that the use of a bridge capture assay for detecting an autoantibody to nephrin is a straightforward, technically uncomplicated approach with superior performance, in particular in terms of sensitivity, compared to comparable state of the art methods.

The present invention is also based on the surprising finding by the inventors that a sensitive detection is possible using a bridge capture assay without carrying out a concentration step.

In a preferred embodiment, the term "bridge capture assay", as used herein, refers to an assay for the detection of an antibody, wherein the detection depends on the formation of a complex comprising a first polypeptide comprising the mammalian, preferably human nephrin or an epitope or variant thereof bound to a first binding site for the antigen on the antibody and a second polypeptide comprising the mammalian, preferably human nephrin or an epitope or variant thereof bound to a second binding site for the antigen on the antibody, followed by detection of said complex. The detection is positive only if this complex is formed, by contrast to a detection method that is positive if only one polypeptide comprising the antigen or an epitope or variant thereof is bound to the antibody to be detected, for example detection via a secondary antibody which is positive no matter whether a second polypeptide comprising the antigen or an immunoreactive variant thereof is bound to the antibody or not. In a more preferred embodiment, the first polypeptide comprising the mammalian, preferably human nephrin or an epitope or variant thereof differs from the second polypeptide comprising the mammalian, preferably human nephrin or an epitope or variant thereof, because the two polypeptides have two different functionalities and functions in the assay. For example, the first polypeptide may be immobilized or configured for immobilization and the second polypeptide may be labeled or configured for labeling. The state of the art describes how bridge capture assays for the detection of an antibody may be set up, for example in Patterson, M. *et al.* (2021) A bridging assay for detection and characterization of anti-drug antibodies to dostarlimab, a new anti-PD-1 therapeutic monoclonal antibody, *AAPS Open* **7** (11). https://doi.org/10.1186/s41120-021-00045-y, and Kikkas, I. et al. (2013) A simple and fast nonradioactive bridging Immunoassay for insulin autoantibodies, PLoS ONE 8(7): e69021.

In a more preferred embodiment, the complex may be detected by immobilization of the complex and detection of the complex using a detectable label. The first polypeptide may be immobilized or configured for immobilization. The second polypeptide may be labeled with a detectable label or configured for labeling. If the immobilized complex is washed and any unbound second polypeptide with a label removed, any antibody bound to the polypeptide may be detected using the detectable label. The complex may be formed after the first polypeptide is immobilized or before it is immobilized. The method may comprise the steps a) contacting the first polypeptide with the antibody in or from the sample, b) immobilizing the first polypeptide, c) contacting the second polypeptide with the antibody in or from the sample, and d) detecting any immobilized complex comprising the first and the second polypeptide. The complex may be washed to remove unbound labeled polypeptide. These steps may be carried out in any order that allows the complex comprising the antibody to be detected and the first and second polypeptide to be formed and detected, for example, but not limited to the order of step b), followed by step c), followed by step a) and finally step d). Step a), followed by b), followed by c) and then d) is another option. Some of these steps may be carried out simultaneously, preferably steps a) and c), followed by steps b) and d).

In another more preferred embodiment, rather than depending on a single detectable label in combination with immobilization, the formation of the complex may be detected using a method detecting the interaction between a first polypeptide comprising the mammalian, preferably human nephrin or an epitope or variant thereof on the one hand and the second polypeptide comprising the mammalian, preferably human nephrin or an epitope or variant thereof. Label-free methods are available to detect this interaction. The method is preferably chosen from the group comprising surface plasmon resonance, mass spectrometry, light scattering and size exclusion chromatography. The person skilled in the art is familiar with these methods, and they are described in F. Lottspeich, J. W. Engels, Bioanalytik, 3rd edition, Springer 2012.

In another more preferred embodiment, the first polypeptide and the second polypeptide may each be labeled such that the labels of the first and the second polypeptide yield a signal only if in close proximity. In a preferred embodiment, the first polypeptide is labeled with a first label and the second polypeptide is labeled with a second label which is a label other than the first label. In another preferred embodiment, the first and the second polypeptide are each labeled with the same label, provided that the two identical label molecules yield a signal if in close proximity. Förster Resonance Energy Transfer (FRET)-capable pairs of fluorescent dyes may be used. The person skilled in the art is familiar with these methods and they are described, for instance, in Saraheimo, S. et al. (2013) Time-Resolved FRET-Based Approach for Antibody Detection - A New Serodiagnostic Concept. PLoS ONE 8(5): e62739. The application for detecting antibodies in a sample is described in Yue, H. et al. (2023) Diagnostic TR-FRET assays for detection of antibodies in patient samples, Cell Reports Methods, (3)3, 100421.

In a preferred embodiment, the first polypeptide is configured for immobilization on a solid carrier. A particularly preferred way for the configuration or the immobilization is modifying the polypeptide such that it comprises an affinity tag or ligand while the carrier comprises the matching ligand or affinity tag, respectively. In a preferred embodiment, the carrier is configured for immobilizing the first polypeptide. The carrier may comprise reactive chemical groups such as thiol, amino, epoxide, ester and anhydride groups that react with reactive side chain groups of the polypeptide. The polypeptide may comprise reactive chemical groups such as thiol, amino, epoxide, ester and anhydride groups that react with reactive chemical groups of the carrier. In a preferred embodiment, the term "immobilized", as used herein, refers to a molecule bound to a solid carrier insoluble in an aqueous solution, more preferably via a covalent or non-covalent bond, electrostatic interactions, encapsulation, unspecific absorption, printing or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilization techniques for microfluidic assays, Biomicrofluidics 7(4), 041501. This way, the immobilized molecule, together with the insoluble carrier, may be separated from an aqueous solution in a straightforward manner, for example by centrifugation or decanting. An immobilized molecule may be immobilized in a reversible or irreversible manner. Various ways to immobilize molecules are described in the literature, for example in Kim, D., and Herr, A. E. (2013), Protein immobilization techniques for microfluidic assays, Biomicrofluidics 7(4), 041501. In addition, various reagents and kits for immobilization reactions are commercially available, for example from ThermoFisher (product numbers 21435, 26196 or 20266).

In a preferred embodiment, the sample comprises a representative set of mammalian, more preferably human IgG, IgA and IgM class antibodies, most preferably IgG class antibodies. It is preferably selected from the group consisting of whole blood, plasma, serum and urine, preferably serum or plasma, most preferably serum. The sample may be a liquid sample or a dried blood spot made using the sample, preferably whole blood, plasma, serum or capillary blood, preferably capillary blood.

The mammalian, preferably human antibody to be detected, referred to as "antibody to nephrin", binds specifically to the mammalian, preferably human (SEQ ID NO: 2) nephrin. In a preferred embodiment, the term "binding specifically", as used herein, preferably means that the binding reaction is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore^{™} equipment at 25 °C in PBS buffer at pH 7 using a protein expressed and purified as described in Example 1.

In a preferred embodiment, the mammalian antibody is a mammalian autoantibody. In a preferred embodiment, the term "autoantibody", as used herein, refers to an antibody which binds specifically to a structure from the mammal, more preferably human which produces said antibody. In a subject suffering from an autoimmune disease associated with the presence of the antibody or more likely to suffer from such an autoantibody disease, the level of such antibody is elevated compared to the level in the average healthy subject. Therefore, an autoantibody is an endogenous molecule produced within a subject's body. Typically, the constant region of a mammalian antibody such as an autoantibody may be composed of a heavy chain constant region (C_{H}) and a light chain constant region (C_{L}). In one embodiment, the mammalian (auto)antibody comprises a C_{H} selected from the group comprising SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 and SEQ ID NO: 23 or a variant thereof, preferably selected from the group comprising SEQ ID NO: 19, SEQ ID NO: 20, SEQ ID NO: 21 and SEQ ID NO: 22 or a variant thereof. In one embodiment, the mammalian (auto)antibody comprises a C_{L} selected from the group comprising SEQ ID NO: 24, SEQ ID NO: 25, SEQ ID NO: 26, SEQ ID NO: 27, SEQ ID NO: 28 and SEQ ID NO: 29 or a variant thereof. More preferably, the autoantibody is a human autoantibody, more preferably a human autoantibody of class IgG, IgM or IgA, preferably IgG. The variable domain is capable of binding specifically to the mammalian, preferably human nephrin. In one embodiment, the constant region binds specifically to molecules recognizing the constant region of IgG class antibodies such as secondary antibodies. It may have sequence elements shared by other antibodies, such as IgG class antibodies, from the same organism. Thus, the autoantibody may have the sequence of an antibody's constant regions from the animal, preferably human, making it, but the variable region is able to bind specifically to the endogenous molecule of the animal, such as mammalian nephrin or a variant thereof.

As the person skilled in the art is aware, an autoantibody is a mixture of polyclonal antibodies. In a preferred embodiment, the term "an autoantibody", as used herein, refers to a specific autoantibody or a mixture of autoantibodies to a certain target. For some applications, it may be useful not to detect the entire population of autoantibodies, but only a subset of them, potentially a single autoantibody present within the population.

In a preferred embodiment, the assay is configured such that the presence of an autoantibody to mammalian, preferably human nephrin is associated with a positive signal and detection of the signal implies that the antibody detected is an antibody to nephrin. In another preferred embodiment, the assay is configured such that the presence of a group of antibodies, preferably to kidney targets, more preferably anti-slit diaphragm antibodies, among them the antibody to nephrin, yields a signal, but this signal only shows that at least one antibody from this group is present and does not show whether or not it is the antibody to nephrin, another antibody or two or more antibodies from said group of antibodies. This may be done by coating a carrier with a mixture of kidney antigens including nephrin and detecting any bound antibody with a mixture of labeled detection molecules, among them a polypeptide comprising an epitope of mammalian, preferably human nephrin, all carrying the same label or labels which cannot be distinguished from each other. In a preferred embodiment, an antibody to kidney targets is selected from the group comprising an antibody to nephrin, an antibody to THSD7a, an antibody to PLA2R, an antibody to Exostosin, an antibody to Kirrel 1 and an antibody to Podocin, more preferably an antibody to Kirrel 1 and an antibody to Podocin and an antibody to Nephrin (Raglianti, V. (2025) Anti-slit Antibodies against Podocin and Kirrel1 in Pediatric and Adult Podocytopathies, J. Am. Soc. Nephrol. doi: 10.1681/ASN.0000000642).

The teachings of the present invention may not only be carried out using the polypeptides, in particular at least one polypeptide comprising the native sequence of at least one epitope of a mammalian, preferably human nephrin or of the mammalian, preferably human nephrin having the exact sequence referred to in this application explicitly, for example by function, name, sequence or accession number, or implicitly, but also using variants of such polypeptides. In preferred embodiments, a polypeptide comprising at least one epitope of a mammalian, preferably human nephrin comprises a polypeptide comprising the entire amino acid sequence of said nephrin. However, in another preferred embodiment, the term also relates to a polypeptide, which does not comprise the entire amino acid sequence, but is truncated at one or both termini, as long as it comprises at least one epitope or immunoreactive variant thereof, as described herein.

In a preferred embodiment, the term "variant", as used herein, may refer to at least one fragment of the full length sequence referred to, more specifically one or more amino acid which is, relative to the full-length sequence, truncated at one or both termini, i.e. the C-terminus, N-terminus or both, by one or more amino acids, respectively. Such a fragment comprises or encodes for a peptide having at least 6, 7, 8, 9, 10, 12, 15, 16, 17, 18, 19, 20, 25, 50, 75, 100, 150, 200, 300, 400, 500, 600, 700, 800, 900, 950 1000, 1010, 1020, 1030, 1040, 1050, 1100, 1200, 1300, 1350, 1360, 1370, 1380, 1390, 1400, 1410, 1420, 1420 or 1430 successive amino acids of the original sequence or a variant thereof. The total length of the variant may be at least 6, 7, 8, 9, 10, 12, 15, 16, 17, 18, 19, 20, 25, 50, 75, 100, 150, 200, 300, 400, 500, 600, 700, 800, 900, 950 1000, 1010, 1020, 1030, 1040, 1050, 1100, 1200, 1300, 1350, 1360, 1370, 1380, 1390, 1400, 1410, 1420 or 1430 or more amino acids.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98, 99, 99.3, 99.4, 99.5, 99.6, 99.7, 99.8 or 99.9, preferably at least 80% or 99% identical to the reference amino acid sequence referred to or the fragment thereof, wherein amino acids other than those essential for the biological activity, for example the ability of an antigen to bind to the mammalian, preferably human antibody, or the fold or structure of the polypeptide are deleted or substituted and/or one or more such essential amino acids are replaced in a conservative manner and/or amino acids are added such that the biological activity of the polypeptide is preserved. In other words, in preferred embodiments the variant maintains the biological activity of the full-length protein of which it is derived. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A. et al. (2007). Clustal Wand Clustal X version 2.0. Bioinformatics, 23, 2947-2948) is used using default setting.

In a preferred embodiment, the polypeptide and variants thereof may comprise a tag, such as an affinity tag, wherein the tag preferably is selected from the group comprising His tag, Dinitrophenyl (DNP), FITC, Digoxigenin, anti-DNP, anti-FITC, anti-Digoxigenin GST tag, E tag, FLAG tag, HA tag, myc tag, V5 tag, S tag, SnoopTag, SpyTag, SofTag, Strep tag, Strep tag II, T7 Epitope tag, immobilized nickel, glutathione, chitin, 18A, biotin tag, ALFA-tag, AviTag, ACP, BCCP, Calmodulin, Chitin binding protein, ELK16, C-Tag, iCap tag, polyglutamate tag, polyarginine tag, NE-tag, Rho1D4-tag, SBP-tag, Softag 1, Softag 3, Spot-tag, TC tag, Ty tag, VSV-tag, TAP tag, thioredoxin, flash, poly aspartate, Isope, maltose binding protein, nus, NE, ProtA, ProtC, Tho1d4 and Xpress tag. Such a tag may be used for a polypeptide that is configured for immobilization or is immobilized.

In some embodiments, the polypeptide comprises one or more than one linker, e.g., between the polypeptide and a label and/or tag for immobilizing the polypeptide on a solid phase. Suitable linkers are commonly known in the art and include peptide or polypeptide sequences that are typically short, e.g., 50 or fewer amino acids long, but may be longer in certain embodiments. In some embodiments, the linker comprises 1, 2, 3, 4, 5, 6, 7, 8, 9, 10, 11, 12, 13, 14, 15, 16, 17, 18, 19, 20, 25, 30, 35, 40, 45, 50 or more amino acids, such as 100, 200 or even more. In some embodiments, the linker is 50 amino acids long or even shorter such as 45, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 amino acid. Typical peptide linker sequences contain Gly, Val and Thr residues. Other near neutral amino acids, such as Ser and Ala can also be used in the linker sequence. Amino acid sequences and other molecules which may be usefully employed as linkers include those disclosed in Murphy et al. (1986) Proc Natl Acad Sci. USA 83:8258-8262; Chen et al. (2013), Adv. Drug. Deliv. Rev. 65(10):1357-1369; Zhang et al., (2020) Biochemistry 59(2):175-178; US4935233 and US4751180. In some embodiments, the linker may be selected from the group comprising Glyₙ, wherein n= 2, 3, 4, 5, 6, 7, 8, 9, 10, 11, 12, 13, 14, 15 or more; AlaₙAspₒProₚ, wherein n=2, 3, 4, 5, 6, 7, 8 or more, o=1, 2, 3, 4 or more and p=1, 2, 3, 4 or more, such as Ala₃AspPro; Hisₙ, wherein n=3, 4, 5, 6, 7, 8, 9, 10, 11, 12, 13, 14 or more; Gly₂ValThr; GlyThrGly₄; AlaSerGly₄SerGly₃; (GlyₙSerₒ)ₚ, wherein n=2, 3, 4, 5, 6, 7, 8, o=1, 2, 3, 4, p=1, 2, 3, 4, 5, 6; Argₙ, wherein n=2, 3, 4, 5, 6; Ala₃ValGluLeuGlu; Gly₄ThrGly₃Ser; GlySer₂Gly. In some embodiments, the linker may be selected from the group comprising amine linker; chloroalkane linker; alkyl chain linker; linker composed of building blocks from beta-alanine, 4-aminobutyric acid, (2-aminoethoxy) acetic acid, 5-aminovaleric acid, 6-aminohexanoic acid, 8-amino-3,6-dioxaoctanoic acid, 12-amino-4,7,10-trioxadodecanoic acid, 15-amino-4,7,10,13-tetraoxapenta-decanoic acid or trioxatridecan-succinamic acid. Other suitable linkers will be apparent to those skilled in the art. Thus, the term linker is not restricted to the examples explicitly mentioned herein. In some embodiments, the linker is cleavable, e.g., by one or more restriction enzymes or proteases, such as by TEV protease, PreScissionTM protease, Thrombin, Factor Xa or enterokinase, or by a chemical reaction. In some embodiments, the linker is not cleavable. In some embodiments, the linker is flexible. In some embodiments, the linker is rigid. The polypeptide may have an N-terminal linker. The polypeptide may have a C-terminal linker. The polypeptide may have an N-terminal linker and a C-terminal linker.

Moreover, variants may also be generated by N- or/and C-terminal fusion of polypeptides, fragments or variants thereof with other known polypeptides or variants thereof or artificial sequences such as linkers and comprise active portions or domains. Any fused sequences are chosen such that the ability of the antigen of the invention or a variant thereof to bind specifically to the antibody to be detected or the diagnostic reliability, in particular sensitivity and/or specificity, is not significantly altered, let alone abolished.

In a preferred embodiment, the term "sensitivity" refers to the number of samples correctly determined as positive relative to the total number of samples examined. In a preferred embodiment, the term "specificity" refers to the number of samples correctly determined as negative relative to the total number of samples examined.

Any variant of a mammalian, preferably human nephrin or epitope thereof has the ability to bind specifically to the mammalian, preferably human antibody binding specifically to the mammalian nephrin as found in a patient suffering from a nephrological autoimmune disease associated with the presence of such antibody in a sample. Exemplary variants comprise SEQ ID NO: 1, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 and SEQ ID NO: 13.

The polypeptide used to carry out the inventive teachings, including any variants, is preferably designed such that it comprises at least one epitope recognized by and/or binding specifically to an antibody to mammalian, preferably human nephrin. In one embodiment, such polypeptide comprises a stretch of 6, 7, 8, 9, 10, 11, 12, 20, 25, 30, 40, 50, 60, 70, 80, 90, 100, 113, 125 or more, preferably at least 9 but no more than 16, consecutive amino acids from mammalian, preferably human nephrin. The person skilled in the art is familiar with guidelines used to design peptides having sufficient immunogenicity, for example those described in Jackson, D. et al. (1999), Preparation and properties of totally synthetic immunogenes, Vaccine 18( 3-4), 355-361; and Black, M. et al. (2010), Advances in the design and delivery of peptide subunit vaccines with a focus on Toll-like receptor agonists, Expert Rev Vaccines, 9(2), 157-173. Briefly, it may be desirable that the peptide meets as many as possible of the following requirements: (a) it has a high degree of hydrophilicity, (b) it comprises one or more residues selected from the group comprising aspartate, proline, tyrosine and phenylalanine, (c) it has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V. et al. (2008), PEPOP: Computational design of immunogenic peptides, BMC Bioinformatics 9:71.

The polypeptide used to carry out the invention, in particular the mammalian nephrin or variant thereof, may be provided in any kind of conformation. In a preferred embodiment, the polypeptide is folded in the sense that the epitope(s) essential for the binding to the inventive antibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably CD spectroscopy is used.

In another preferred embodiment, the carrier such as the diagnostically useful carrier, is a bead. Preferably, the bead is provided in the form of an aqueous suspension having a bead content of from 10 to 90%, preferably from 20 to 80%, preferably from 30 to 70%, more preferably from 40 to 60% (w/w). The person skilled in the art is familiar with such beads (Diamindis, E. P., Chriopoulus, T. K., Immunoassays, 1996, Academic Press), which are commercially available, for example Bio-Plex COOH beads MC10026-01 or 171-506011 from Bio-Rad.

In another preferred embodiment, the carrier is a microtiter plate comprising a multitude of wells such as at least 8 wells that may be used for ELISA. At least 3, preferably 4, more preferably 5, or even more calibrators, at defined concentrations may be used to set up a calibration curve for semi-quantitative analysis. When the inventive method is carried out, the calibrators, which typically cover a range of concentrations covering the calibrating curve, may be processed and developed in parallel to the samples. A human monoclonal antibody may be used as a calibrator, for example clone B-12 from Santa Cruz, product number sc-377246, may be used.

A label, also referred to as detectable label is used to label a polypeptide or provided if the polypeptide is configured for labeling. It is preferably selected from the group comprising a fluorescent, a radioactive, a chemiluminescent label, a heavy metal such as gold label, a nanoparticle, a bead or an enzymatically active label, preferably one catalyzing a colorimetric or chemiluminescent reaction. In a preferred embodiment, a fluorescent label is selected from the group comprising Alexa dyes, FITC, TRITC and green fluorescent protein (GFP). Iodine-125 may be used as radioactive label. In a preferred embodiment, an enzymatically active label is selected from the group comprising horseradish peroxidase, glucose oxidase, beta galactosidase, alkaline phosphatase and luciferase. In a preferred embodiment, a chemiluminescent label is selected from the group comprising luminol or a derivative, an acridinium ester and luciferase. The person skilled in the art is able to choose suitable labels and to attach them to proteins, nucleic acids and other molecules (Hassanzadeh, L., Chen, S., Veedu, R. N. (2018) Radiolabeling of Nucleic Acid Aptamers for Highly Sensitive Disease-Specific Molecular Imaging. Pharmaceuticals (Basel). 2018;11(4):106. Published 2018 Oct 15. doi: 10.3390/ph11040106 and Hermanson, G. T. Bioconjugate Techniques, 3rd Edition (2013*)* and Obermaier, C. et. al. (2015) Principles of protein labeling techniques. Methods Mol Biol. 1295: 153-65), and a wide range of labeled molecules are commercially available.

In a preferred embodiment, a ligand to an affinity tag, as used herein, is an artificial entity binding specifically to an affinity tag, typically a chemically synthesized modification or a recombinant protein or peptide attached to a molecule of interest. The ligand to an affinity tag depends on the type of affinity tag chosen and may be selected from the group consisting of His, Dinitrophenyl (DNP), FITC, Digoxigenin, anti-DNP, anti-FITC, anti-Digoxigenin, immobilized nickel, glutathione, chitin, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV, biotin, Xpress Tag and a recombinant antibody binding to the ligand to an affinity tag. A solid carrier configured for the immobilization of a polypeptide may carry a ligand. For example, the polypeptide may comprise avidin or derivative thereof and the carrier may comprise a biotin or *vice versa.*

Various methods or uses according to the invention can be conducted with a sample from a subject or patient as described herein. These methods or uses can also be characterized as *"in vitro"* methods or *"in vitro"* uses.

In a preferred embodiment, the term "chemical solution reactive with a detectable label" refers to a compound which, upon exposure to the detectable label in a liquid, emits a detectable signal. The solution may comprise a chromogenic substrate of an enzymatically active label. For example, 3,3', 5,5' tetramethylbenzidine (TMB)/H₂O₂ may be used if the label is a peroxidase. The solution may comprise a small inorganic or organic compound capable of reacting with a chemiluminescent label. In the case of an acridinium ester, a mixture of H₂O₂ and sodium hydroxide is frequently used as the chemical solution. Various other chemical solutions and detectable labels are known in the art (Weeks, I., Beheshti, I., McCapra, F., Campbell, A. K., Woodhead, J. S. (1983) Acridinium esters as high specific activity labels in immunoassay. Clin Chem 29: 1474-1479), Thermo Scientific Pierce Antibody Production and Purification Technical Handbook, Version 2, www.thermoscientific.com).

In a preferred embodiment, the methods, uses, mammalian antibodies, carriers and kits described herein are for diagnosing a disease and/or for aiding in the diagnosis of a disease. In a preferred embodiment, the term "diagnosis", as used herein, is to be understood in its broadest possible sense and may refer to any kind of procedure aiming to obtain information instrumental in the assessment whether a patient, known or an anonymous subject from a cohort, suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs, or to find out whether a sample is from such a patient. Such information may be used for a clinical diagnosis but may also be obtained by an experimental and/or research laboratory for the purpose of general research, for example to determine the proportion of subjects suffering from the disease in a patient cohort or in a population. Such information may also be used to determine whether a sample is or is not a sample from a subject who is more likely than an average healthy subject to suffer, have suffered or suffer in the future from a nephrological autoimmune disease. Alternatively, a person who or a sample of whom is the subject of research may be diagnosed, for example to find out whether this patient is a suitable subject for the study, for example as a part of a cohort of subjects suffering from a disease or as a test subject, for example for testing the efficacy of a candidate drug. In a preferred embodiment, patients suffering from an autoimmune disease, preferably a nephrological autoimmune disease such as FSGS are selected for inclusion in a cohort for a clinical study aiming to test the efficacy of immunosuppressive drugs, preferably systemic immunomodulators. For example, if a patient has such autoantibodies, they and their samples may be included into the cohort, because there is the potential that the autoimmune disease may be ameliorated by administration of immunosuppressive drugs, preferably systemic immunomodulators. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient. Thus, in some embodiments, the person to be diagnosed, *i.e.,* the "subject" or "patient", is an anonymous blood donor whose blood may be donated or used to obtain therapeutically or diagnostically useful antibodies. The term "diagnosis" also refers to negative diagnosis, *i.e.,* the case where a mammalian antibody to a mammalian, preferably human nephrin are absent in a sample from a patient. In these embodiments, the absence indicates that the patient may suffer from a disease other than a disease associated with the presence of said antibodies. In another preferred embodiment, the detection of a mammalian, preferably human antibody to mammalian, preferably human nephrin is considered to imply a definitive diagnosis of a nephrological autoimmune disease because of the presence of the antibody.

Thus, in a preferred embodiment, the term "diagnosis", "diagnosing" or "diagnostic" or similar terms encompasses diagnosis, prognosis, theragnosis and monitoring in an autoimmune disease, preferably a nephrological autoimmune disease, such as those described herein. As used herein, the term "theragnosis" refers to the identification, for example by diagnostic methods, of patients who might benefit from a particular therapy and, optionally, the subsequent treatment of said patients, for example by administration of an immunosuppressive drug.

In a preferred embodiment, the methods and products according to the present invention may be used for interaction studies, including determining whether a drug candidate or other compound may interfere with the binding of a mammalian antibody binding specifically to a slit membrane protein or may affect any downstream process or the strength of its binding to its target. In a preferred embodiment, they may be used for monitoring the immune response, more preferably the emergence and/or titer of antibodies to a mammalian, preferably human nephrin, following the administration of an immunogenic composition comprising at least one polypeptide comprising at least one epitope of a mammalian, preferably human nephrin or an immunogenic variant thereof, for example to a mammal, which may be a mammal other than a human such as a laboratory animal.

In a preferred embodiment, the level of the antibody to mammalian, preferably human nephrin may be monitored using the method according to the present invention. It may be monitored over a period of at least 1, 2, 3, 4, 6, 8, 12, 16, 20, 24, 36, 52 or 104 weeks. This may help evaluate the progression of a nephrological autoimmune disease associated with an antibody to nephrin or the response to a treatment, for example by administration of a drug such as an immunosuppressive drug or a kidney transplant. If the level of the antibody increases, this may indicate a high likelihood that the disease deteriorates. It may also indicate rejection of a kidney transplant.

In another preferred embodiment, the methods and products according to the present invention may be used for determining the concentration of a mammalian, preferably human antibody. In a more preferred embodiment, said antibody is a mammalian antibody from a patient suffering from an autoimmune disease such as a nephrological autoimmune disease. In a more preferred embodiment, such a concentration needs to be determined for the purposes of research, for the preparation or for monitoring the quality of reagents, animal models or devices that may or may not be used for the diagnosis of a disease or for aiding in such diagnosis.

In many cases the mere detection of the antibody, in other words determining whether or not detectable levels of the antibody are present in the sample, is sufficient for the diagnosis. In another embodiment, this may involve determining whether the concentration is at least 10%, preferably 20%, 50%, 100%, 200%, 500%, 1000%, 2000%, 2500%, 5000%, 10000%, 20000%, 50000%, 100000%, 1000000% or 10000000% times higher than the concentration of the antibody of interest found in the average healthy subject. If the antibody can be detected, this may indicate an increased likelihood that the patient suffers, will suffer, or has suffered from the disease compared to an average healthy subject.

The person skilled in the art will appreciate that a clinician does usually not arrive at the conclusion whether or not the patient suffers or is likely to suffer from a disease, condition or disorders solely on the basis of a single diagnostic parameter, but needs to take into account other aspects, for example the presence of other antibodies, markers, blood parameters, clinical assessment of the patient's symptoms or the results of medical imaging or other non-invasive methods, to arrive at a conclusive diagnosis. See Baenkler H. W. (2012), General aspects of autoimmune diagnostics, in Renz, H., Autoimmune diagnostics, 2012, de Gruyter, page 3. In a preferred embodiment, the meaning of any symptoms or diseases referred to throughout this application is in line with the person skilled in the art's understanding as of the filing date or, preferably, earliest priority date of this application as evidenced by textbooks and scientific publications. In a preferred embodiment, the methods or uses or products are not used, taken alone, to arrive at a definite, final diagnosis.

In a preferred embodiment, any information or data demonstrating the presence or absence of the antibody may be communicated to the patient or a medical doctor treating the patient orally, preferably by telephone, in a written form, preferably fax or letter, or in an electronic form *via* fax or *via* the internet, for example as an email or text message.

An assay for detecting the presence of antibodies to nephrin is an important tool in the context of diagnostics and research in the field of nephrological autoimmune disease. In a preferred embodiment, the term "nephrological autoimmune disease associated with the presence antibodies to nephrin", as used herein, refers to a disease related to the function of kidneys, wherein a certain concentration range of antibodies to mammalian, preferably human nephrin detectable in a sample from a patient, preferably their presence and/or absence, aids a clinician in diagnosing of the disease.

In a preferred embodiment, the disease is minimal change disease (MCD). MCD is characterized by diffuse loss of podocyte foot processes vacuolation and growth of microvilli on the visceral epithelial cells, and this is the cause for symptoms such as proteinuria, edema, weight gain and hypoalbuminemia. Immunosuppressive treatment based on drugs such as corticosteroids is the treatment of choice, alternatively cyclosporin, calcineurin inhibitors, mycophenolate mofetil or rituximab is regularly administered.

In another preferred embodiment, the disease is FSGS. This disease is characterized by scarred glomeruli and damage to renal podocytes, resulting in a decreased filtering function of the kidney and a nephrotic syndrome. Symptoms include proteinuria and edema. FSGS is traditionally diagnosed by renal biopsy, but the detection of antibodies to nephrin may aid in the diagnosis of autoimmune FSGS. FSGS may be treated with immunosuppressants. A significant number of patients with FSGS require a kidney transplant. In patients with autoimmune FSGS, there is the danger that the transplant will fail because the immune system will attack the transplant and destroy it within a few weeks. Therefore, it is helpful to understand whether FSGS is associated with antibodies or not.

In another preferred embodiment, the nephrological disease is a nephrotic syndrome. This disease is characterized by proteinuria, hypoalbuminemia, hyperlipidemia and edema. The nephrotic syndrome may be an idiopathic nephrotic syndrome, i.e. its cause is unknown.

An assay for detecting the presence of antibodies to nephrin is an important tool in the context of diagnostics and research in the field of kidney transplantation. A significant number of patients with a nephrological disease require a kidney transplant. If the nephrological disease is associated with the presence of an antibody to nephrin, for example autoimmune FSGS, there is the danger that the transplant will fail because the immune system will attack the transplant and destroy it within a few weeks. Therefore, it is helpful to understand whether the nephrological disease is associated with an antibody such as an antibody to nephrin. By contrast, if the nephrological disease is not associated with an antibody to nephrin, for example, because it is associated with another, optionally temporary cause such as a toxic drug, this may indicate an increased likelihood that the kidney transplantation will be successful. Immunosuppressive treatment may be in order if a kidney transplant is received by a patient suffering from a nephrological autoimmune disease.

In a preferred embodiment, the method according to the present invention is performed in order to distinguish an autoimmune cause of a nephrological disease, preferably the presence of an antibody to mammalian, preferably human nephrin, from another cause, preferably one or all selected from the group comprising an infection such as AIDS or hepatitis, an allergic reaction, drugs such as nonsteroidal anti-inflammatory drugs and genetic causes, or to identify the autoimmune disease. Autoimmune diseases associated with nephrological diseases and symptoms include Diabetes, Sjögrens's Syndrome, Membranous Nephropathy and Lupus erythematosus.

The inventive teachings, such as the methods, uses, antibodies, carriers and kits described herein, may also be used in a method for preventing or treating a disease, preferably after a diagnosis according to the present invention, comprising the steps a) reducing the concentration of mammalian, preferably human antibodies to nephrin in the subject's blood and/or b) administering one or more immunosuppressive pharmaceutical substances, preferably selected from the group consisting of rituximab, prednisone, methylprednisolone, cyclophosphamide, mycophenolate mofetil, intravenous immunoglobulin, tacrolimus, cyclosporin, methotrexate and azathioprine.

According to the present invention, the presence of an antibody may be detected in a qualitative or a quantitative manner. In a preferred embodiment, the term "detecting in a quantitative manner", as used herein, means that not only the presence of an antibody is detected, but that a result is obtained that includes information regarding the absolute or relative amount of the antibody in the sample. In a more preferred embodiment, a value representing an absolute concentration is obtained. In another more preferred embodiment, a value representing a relative concentration or change of concentration is obtained. In another preferred embodiment, also referred to as "semi-quantitative" approach, the concentration of the antibody is placed in one of several groups, most preferably a concentration window meaning that it is virtually absent, a concentration window meaning that a borderline result is obtained and a concentration window meaning that the antibody is present. A further distinction into categories such as "weak positive" or "strong positive" signal is possible.

The term "isolated" as used herein means that the molecule referred to as being isolated is free or essentially free of at least one component as it is found in nature and/or is separated from its environment as found in nature or from parts thereof. For example, the molecule, such as the first polypeptide or the second polypeptide comprising mammalian, preferably human nephrin or an epitope thereof, may be free or essentially free from some or all components as it is found in its natural environment. Such components may comprise, for example in the case of a mammalian antibody, erythrocytes, leucocytes, thrombocytes, plasma, proteins, nucleic acids, salts, lipids, and nutrients.

According to the present invention, a polypeptide, such as the at least one polypeptide comprising at least one epitope of a mammalian, preferably human nephrin or a variant thereof, may be a recombinant polypeptide. In a preferred embodiment, the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, a polypeptide provided or used according to the present invention such as a polypeptide comprising at least one epitope of a mammalian, preferably human nephrin or a variant thereof or an antibody is an isolated polypeptide, wherein the term "isolated" may mean that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection.

According to the present invention, a kit is provided, comprising a first polypeptide comprising an epitope from the extracellular domain of a mammalian, preferably human nephrin and a second polypeptide comprising an epitope from the extracellular domain of a mammalian, preferably human nephrin. The first polypeptide comprises a mammalian nephrin characterized by SEQ ID NO: 1, preferably a nephrin selected from the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, more preferably SEQ ID NO: 2 or an immunoreactive variant or fragment thereof. The second polypeptide comprises a mammalian nephrin characterized by SEQ ID NO: 1, preferably a nephrin selected from the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, more preferably SEQ ID NO: 2 or an immunoreactive variant or fragment thereof. In a preferred embodiment, the first polypeptide and/or the second polypeptide does not include an epitope from mammalian, preferably human nephrin which is not on the extracellular domain. In a preferred embodiment, no epitope from mammalian, preferably human nephrin which is not on the extracellular domain is present when the first polypeptide is coated and/or when the sample is contacted with the first polypeptide. In a preferred embodiment, at least one of, preferably both the first polypeptide comprising mammalian nephrin, preferably human nephrin or an epitope there and the second polypeptide comprising mammalian nephrin, preferably human nephrin or an epitope there does/do not comprise an epitope from the mammalian, preferably human nephrin other than an epitope from the extracellular domain. Any polypeptide immobilized or configured for immobilization may comprise two or more separate polypeptides which together form at least one epitope from the extracellular domain of mammalian, preferably human nephrin. It is essential that at least one polypeptide that is immobilized or configured for immobilization has at some point been isolated and/or purified to remove non-specifically binding cellular components. Preferably both the first and second polypeptide have been purified and/or isolated.

In a preferred embodiment, the kit comprises instructions for use which describe at least one way of carrying out the assay for detecting a mammalian antibody to a mammalian, preferably human nephrin in an assay, preferably for the diagnosis of or for aiding in the diagnosis of a disease.

In a preferred embodiment, any method or use according to the present invention may be intended for a non-diagnostic use, i.e. determining the presence of a mammalian antibody to mammalian, preferably human nephrin, for a use other than diagnosing a patient. For example, the method or use may be for testing *in vitro* the efficiency of a medical device designed to remove a mammalian, preferably human antibody from a patient's blood, wherein the testing is performed on a liquid other than patient's blood. After the use of the medical device with a patient, its capacity to remove the antibody may be checked by running a solution comprising an antibody, preferably a standard solution which may comprise a recombinant and/or purified and/or monoclonal antibody to a mammalian, preferably human nephrin through the device, followed by use of the method according to the present invention to confirm that less or no antibody is in the solution that has been passed through the device, i.e. showing that the device has still the capacity to remove antibody from the solution.

According to the present invention, the method or use may be used for testing the efficacy of a drug candidate which may be used to treat patients suffering from or likely to suffer from a nephrological autoimmune disease. Such a drug candidate may be any molecule capable of interfering with the interaction between the mammalian, preferably human nephrin and the antibody binding to it.

In another preferred embodiment, the method may be for calibrating, standardizing or confirming the reliability of an analytical and/or diagnostic assay, e.g., for the diagnosis of a disease associated with the presence of a mammalian antibody to a mammalian, preferably human nephrin, and may involve detecting an antibody to mammalian, preferably human nephrin in a solution, which is not a sample from a patient who requires a diagnosis but is known to comprise an antibody to mammalian, preferably human nephrin, preferably at a known concentration. For example, it may be a recombinant antibody or a sample diluted in a dilution buffer such as PBS from an anonymous patient whose identity cannot be traced back. Alternatively, the solution may be a negative control. Such method may be run in parallel with, after or before analytical and/or diagnostic method.

The term "at least one" comprises "exactly one" and "more than one". For example, a polypeptide comprising at least one epitope may refer to a polypeptide comprising exactly one epitope but it may also refer to a polypeptide comprising more than one epitope, such as 2, 3, 4, 5, 6, 7, 8, 9, 10, 11, 12, 13, 14, 15, 20, 25, 30, 35, or even more epitopes. In some embodiments, the term "one or more" comprises "at least one". In some embodiments, "one or more" and "at least one" are interchangeable.

In a preferred embodiment, the method according to the invention does not comprise a step aiming to concentrate autoantibodies to be detected in a sample, preferably from the group comprising precipitating, preferably immunoprecipitating, concentrating using a filter device, immobilizing existing antibodies present in a sample. In a more preferred embodiment, the term "concentrating", as used herein, means that the concentration of the antibodies is increased at least by at least 20, 30, 40, 50, 60, 70, 80, 90, 95, 100, 150, 200, 300, 400, 500 of 1000%, based on the volumes before and after the step that it specifically designed to concentrate the antibodies. The person skilled in the art is familiar with suitable methods such as ammonium sulphate concentration or ion exchange chromatography which are described, for example, in Krisna C. Duong-Ly, Sandra B. Gabelli, Chapter Seven - Salting out of Proteins Using Ammonium Sulfate Precipitation, Editor(s): Jon Lorsch, Methods in Enzymology, Academic Press, Volume 541, 2014, Pages 85-94. Suitable devices are commercially available, for example Amicon (product number UFC5010) or Centricon (exemplary product number: UFC710008) devices from SigmaAldrich.

In some embodiments, the term "preferably" comprises "optionally". In some embodiments, "preferably" and "optionally" are interchangeable.

The present invention is further illustrated by the following non-limiting examples from which further features, embodiments, aspects and advantages of the present invention may be taken. The present invention comprises a range of novel nucleic acid and amino acid sequences, in particular
**SEQ ID NO2 (extracellular domain of human nephrin):**

MALGTTLRASLLLLGLLTEGLAQLAIPASVPRGFWALPENLTVVEGASVELRCGVSTPGSAV
QWAKDGLLLGPDPRIPGFPRYRLEGDPARGEFHLHIEACDLSDDAEYECQVGRSEMGPELV
SPRVILSILVPPKLLLLTPEAGTMVTWVAGQEYVVNCVSGDAKPAPDITILLSGQTISDISANVN
EGSQQKLFTVEATARVTPRSSDNRQLLVCEASSPALEAPIKASFTVNVLFPPGPPVIEWPGL
DEGHVRAGQSLELPCVARGGNPLATLQWLKNGQPVSTAWGTEHTQAVARSVLVMTVRPED
HGAQLSCEAHNSVSAGTQEHGITLQVTFPPSAIIILGSASQTENKNVTLSCVSKSSRPRVLLR
WWLGWRQLLPMEETVMDGLHGGHISMSNLTFLARREDNGLTLTCEAFSEAFTKETFKKSLIL
NVKYPAQKLWIEGPPEGQKLRAGTRVRLVCLAIGGNPEPSLMWYKDSRTVTESRLPQESRR
VHLGSVEKSGSTFSRELVLVTGPSDNQAKFTCKAGQLSASTQLAVQFPPTNVTILANASALR
PGDALNLTCVSVSSNPPVNLSWDKEGERLEGVAAPPRRAPFKGSAAARSVLLQVSSRDHG
QRVTCRAHSAELRETVSSFYRLNVLYRPEFLGEQVLVVTAVEQGEALLPVSVSANPAPEAFN
WTFRGYRLSPAGGPRHRILSSGALHLWNVTRADDGLYQLHCQNSEGTAEARLRLDVHYAPT
IRALQDPTEVNVGGSVDIVCTVDANPILPGMFNWERLGEDEEDQSLDDMEKISRGPTGRLRI
HHAKLAQAGAYQCIVDNGVAPPARRLLRLVVRFAPQVEHPTPLTKVAAAGDSTSSATLHCRA
RGVPNIVFTWTKNGVPLDLQDPRYTEHTYHQGGVHSSLLTIANVSAAQDYALFTCTATNALG
SDQTNIQLVSISRPDPPSGLKVVSLTPHSVGLEWKPGFDGGLPQRFCIRYEALGTPGFHYVD
VVPPQATTFTLTGLQPSTRYRVWLLASNALGDSGLADKGTQLPITTPGLHQPSGEPEDQLPT
EPPS

### Bridge-ELISA/ELISA:

**SEQ ID NO12: (extracellular domain of human nephrin with C-terminal His tag)** 
MALGTTLRASLLLLGLLTEGLAQLAIPASVPRGFWALPENLTVVEGASVELRCGVSTPGSAV
QWAKDGLLLGPDPRIPGFPRYRLEGDPARGEFHLHIEACDLSDDAEYECQVGRSEMGPELV
SPRVILSILVPPKLLLLTPEAGTMVTWVAGQEYVVNCVSGDAKPAPDITILLSGQTISDISANVN
EGSQQKLFTVEATARVTPRSSDNRQLLVCEASSPALEAPIKASFTVNVLFPPGPPVIEWPGL
DEGHVRAGQSLELPCVARGGNPLATLQWLKNGQPVSTAWGTEHTQAVARSVLVMTVRPED
HGAQLSCEAHNSVSAGTQEHGITLQVTFPPSAIIILGSASQTENKNVTLSCVSKSSRPRVLLR
WWLGWRQLLPMEETVMDGLHGGHISMSNLTFLARREDNGLTLTCEAFSEAFTKETFKKSLIL
NVKYPAQKLWIEGPPEGQKLRAGTRVRLVCLAIGGNPEPSLMWYKDSRTVTESRLPQESRR
VHLGSVEKSGSTFSRELVLVTGPSDNQAKFTCKAGQLSASTQLAVQFPPTNVTILANASALR
PGDALNLTCVSVSSNPPVNLSWDKEGERLEGVAAPPRRAPFKGSAAARSVLLQVSSRDHG
QRVTCRAHSAELRETVSSFYRLNVLYRPEFLGEQVLVVTAVEQGEALLPVSVSANPAPEAFN
WTFRGYRLSPAGGPRHRILSSGALHLWNVTRADDGLYQLHCQNSEGTAEARLRLDVHYAPT
 
IRALQDPTEVNVGGSVDIVCTVDANPILPGMFNWERLGEDEEDQSLDDMEKISRGPTGRLRI
HHAKLAQAGAYQCIVDNGVAPPARRLLRLVVRFAPQVEHPTPLTKVAAAGDSTSSATLHCRA
RGVPNIVFTWTKNGVPLDLQDPRYTEHTYHQGGVHSSLLTIANVSAAQDYALFTCTATNALG
SDQTNIQLVSISRPDPPSGLKVVSLTPHSVGLEWKPGFDGGLPQRFCIRYEALGTPGFHYVD
VVPPQATTFTLTGLQPSTRYRVWLLASNALGDSGLADKGTQLPITTPGLHQPSGEPEDQLPT
EPPSLEHHHHHHHH
**SEQ ID NO13 (extracellular domain of human nephrin with C-terminal His tag and avid tag for biotinyliation)**

MALGTTLRASLLLLGLLTEGLAQLAIPASVPRGFWALPENLTVVEGASVELRCGVSTPGSAV
QWAKDGLLLGPDPRIPGFPRYRLEGDPARGEFHLHIEACDLSDDAEYECQVGRSEMGPELV
SPRVILSILVPPKLLLLTPEAGTMVTWVAGQEYVVNCVSGDAKPAPDITILLSGQTISDISANVN
EGSQQKLFTVEATARVTPRSSDNRQLLVCEASSPALEAPIKASFTVNVLFPPGPPVIEWPGL
DEGHVRAGQSLELPCVARGGNPLATLQWLKNGQPVSTAWGTEHTQAVARSVLVMTVRPED
HGAQLSCEAHNSVSAGTQEHGITLQVTFPPSAIIILGSASQTENKNVTLSCVSKSSRPRVLLR
WWLGWRQLLPMEETVMDGLHGGHISMSNLTFLARREDNGLTLTCEAFSEAFTKETFKKSLIL
NVKYPAQKLWIEGPPEGQKLRAGTRVRLVCLAIGGNPEPSLMWYKDSRTVTESRLPQESRR
VHLGSVEKSGSTFSRELVLVTGPSDNQAKFTCKAGQLSASTQLAVQFPPTNVTILANASALR
PGDALNLTCVSVSSNPPVNLSWDKEGERLEGVAAPPRRAPFKGSAAARSVLLQVSSRDHG
QRVTCRAHSAELRETVSSFYRLNVLYRPEFLGEQVLVVTAVEQGEALLPVSVSANPAPEAFN
WTFRGYRLSPAGGPRHRILSSGALHLWNVTRADDGLYQLHCQNSEGTAEARLRLDVHYAPT
IRALQDPTEVNVGGSVDIVCTVDANPILPGMFNWERLGEDEEDQSLDDMEKISRGPTGRLRI
HHAKLAQAGAYQCIVDNGVAPPARRLLRLVVRFAPQVEHPTPLTKVAAAGDSTSSATLHCRA
RGVPNIVFTWTKNGVPLDLQDPRYTEHTYHQGGVHSSLLTIANVSAAQDYALFTCTATNALG
SDQTNIQLVSISRPDPPSGLKVVSLTPHSVGLEWKPGFDGGLPQRFCIRYEALGTPGFHYVD
VVPPQATTFTLTGLQPSTRYRVWLLASNALGDSGLADKGTQLPITTPGLHQPSGEPEDQLPT
EPPSLEGSGSGSHHHHHHHHGSGLNDIFEAQKIEWHE

### Examples

### Recombinant expression of full length human nephrin and the extracellular domain of human nephrin (in HEK293T-cells):

cDNA encoding the entire human nephrin or the extracellular domain of nephrin was cloned and ligated into the pTriEx-1 vector (Merck, Darmstadt, Germany) for expression in mammalian cells using standard molecular biology techniques. Two versions of the extracellular domain were prepared, one expressing human nephrin extracellular domain fused to an His-tag (SEQ ID NO: 12) and one expressing the extracellular domain comprising a His-tag and a biotinylation sequence (SEQ ID NO: 13). In order to biotinylate nephrin-H8-avi *in vivo,* the BirA gene from E. coli K12 strain was inserted into pTriEx-1 vector (Merck, Darmstadt, Germany). *In vivo* biotinylation of nephrin-H8-avi was conducted by coexpression of BirA enzyme and nephrin in HEK293T cells.

For transient expression, HEK293T suspension culture in BalanCD-HEK (Irvine Scientific, Fujifilm, catalog number 91165) with 4 mM glutamine was transfected using PEI MAXTM (Polysciences, catalog number 24765) according to the instruction of the manufacturer. For biotinylation of nephrin-H8-avi by coexpressed BirA 2 µg/ml D(+)-biotin (Carl Roth, catalog number 3822) was added to the culture medium.

The cell free cell culture supernatant was collected at day 5 after transfection and used for protein purification.

For the production of immunofluorescence substrates with full length nephrin, the cells were grown in DMEM with 10% fetal calf serum on cover slides and transfected using PEI MAXTM (Polysciences, catalog number 24765) according to the instruction of the manufacturer. At day 3 after transfection the cells were fixed using formalin. For other purposes cells were harvested 5 days after transfection and lysed by high pressure. The lysates were stored in aliquots at - 80 °C until further use.

### Purification of the extracellular domain of human nephrin from HEK293T cell culture supernatant:

The procedure for the purification of the human nephrin extracellular domain fused to an His-tag (SEQ ID NO: 12) or the combination of a His tag and a biotinylation sequence (SEQ ID NO: 13) was identical. First, cell free cell culture supernatant was adjusted to 20 mM 3-morpholinopropane sulfonic acid pH 7.0, 300 mM sodium chloride, 20 mM imidazole, 50 mM magnesium chloride, 0.1 % Triton-X-100 and cleared by centrifugation for60 minutes at 17,600 x g at 4°C. The supernatant was applied to Nickel Agarose Extrachel (ABT Agarose Bead Technologies, Madrid, Spain) equilibrated with 10 mM 3-morpholinopropane sulfonic acid pH 7.0, 300 mM sodium chloride, 20 mM imidazole and eluted by increasing the imidazole concentration to 250 mM. All fractions containing the protein to be purified were pooled, the buffer adjusted to 20 mM 2-(4-(2-hydroxyethyl)-1-piperazinyl) ethanesulfonic acid (HEPES), 250 mM sodium chloride, concentrated by ultrafiltration (VivaSpin, Sartorius, Göttingen, Germany) and stored at -80°C until further use.

For evaluating the protein purity, the final preparation was treated with or without 16 mM dithiotreitol (DTT) and incubated at 70°C for 10 minutes, followed by SDS gel electrophoresis and Coomassie staining. Protein identity was verified by mass spectrometry.

### Example 1: Detection of human antibodies to the extracellular domain of nephrin using ELISA

Washing and sample buffers used for the implementation of the nephrin-ELISA are available for commercial EUROIMMUN ELISA-Test-Kits and may be obtained by ordering product number El 2260-9601 G.

For coating of microtiter plates, the human recombinant extracellular domain of nephrin (SEQ ID NO: 12) was purified as described above.

The purified protein was diluted to 1 µg/ml in phosphate buffered saline (PBS) and 100 µl per well were incubated on a 96-well *Lockwell* C8 *Maxisorp* plate (Thermo Scientific TM, Catalog number 446470) for 2 h at room temperature. After aspiration of the protein solution, each well was washed three times with 200 µL of washing buffer and blocked using 0.1 % (w/v) casein blocking buffer (10 mM phosphate buffer, 145 mM sodium chloride, 0.09 % sodium azide, 8.7 mM Tris, 0.1 % casein, pH 7.4) for 1 h. Following another intensive washing step, 100 µl of samples diluted 1:10 in 0.1 % (w/v) casein blocking buffer were applied to the microtiter plate coated with nephrin. As positive control, an anti-nephrin antibody (anti-nephrin clone B-12 by Santa Cruz, product number sc-377246) was used in three different dilutions (K1 = 1:4.000; K2 = 1:8.000, K3 = 1:16.000). After an incubation for 1 h at room temperature, the samples were aspirated, and the wells were washed three times with 200 µL of washing buffer.

Autoantibody detection was performed as described for commercial EUROIMMUN ELISA Test-Kits (e.g. product number El 2260-9601 G), using 100 µL of peroxidase-labelled anti-human IgG conjugate per well with an incubation for 30 min at room temperature. The wells were washed three time with 200 µL of washing buffer and the color reaction was initiated by addition of 100 µl chromogen/substrate solution (TMB/H₂O₂). Following 15 min incubation at room temperature, the stop solution (0.5 M sulfuric acid) was added and the optical density was measured at 450 nm against 620 nm as a reference.

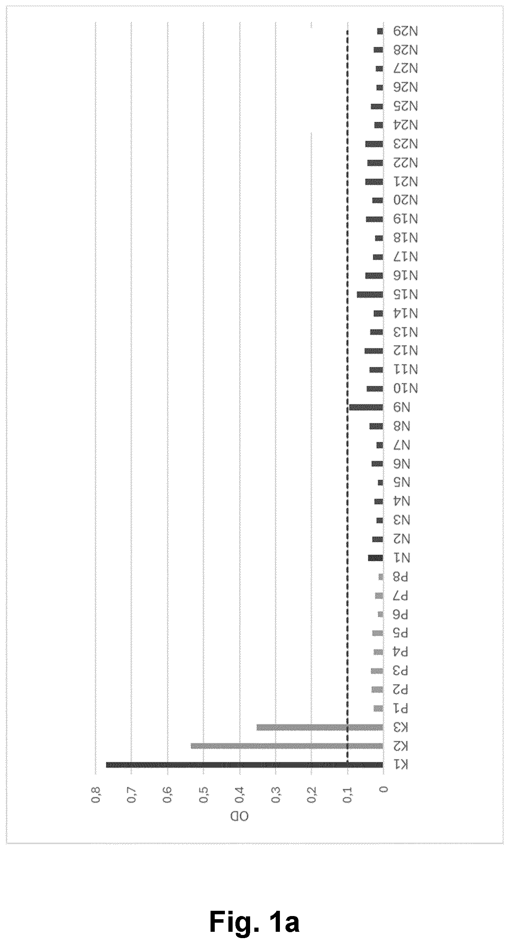

**Fig. 1a** shows the results with samples obtained from 29 healthy blood donors (N1-N29) and 8 samples from patients suffering from minimal change disease which were known to comprise autoantibodies to nephrin (P1-P8). Using the ELISA, none of the positively characterized samples could be distinguished from the negative controls using a cutoff calculated from mean ± five times standard deviation of the signals from all healthy donors.

### Example 2: Detection of human antibodies to the extracellular domain of human nephrin using bridge capture assay

Buffers used for the implementation of the bridge capture assay for detecting antibodies to nephrin are available for commercial EUROIMMUN ELISA-Test-Kits and may be obtained my ordering product number El 2260-9601 G.

For coating of microtiter plates, the human recombinant extracellular domain of nephrin (SEQ ID NO: 12) purified as described in Example 1 was diluted to 0.5 µg/ml in PBS and 100 µL per well were incubated on a 96-well Lockwell C8 Maxisorp plate (Thermo Scientific^{™}, Catalog number 446470) for 2 h at room temperature. After aspiration of the protein solution, each well was washed three times with 200 µL of washing buffer and blocked using 0.1 % (w/v) casein blocking buffer for 1 h. Following another washing step, 25 µl of samples diluted 1:10 in sample buffer containing 0.1 % casein were applied to the microtiter plate. As a positive control, an anti-nephrin antibody was used in three different dilutions (K1-K3) again. After an incubation for 1 h at room temperature, the samples were aspirated and the wells were washed three times with 200 µL of washing buffer.

For autoantibody detection, the human recombinant extracellular domain of nephrin carrying a C-terminal biotinylated avi-tag (SEQ ID NO: 13) was expressed and purified as described with regard to the coated antigen in Example 1 and diluted to 10 µg/ml in PBS containing 0.1 % casein and 100 µL per well were applied. Following an incubation for 30 min at room temperature, the wells were aspirated and washed three times with 200 µL washing buffer.

For detecting the biotinylated nephrin Streptavidin-PolyHRP80 (SDT (Stereospecific Detection Technologies GbR), SP80C; 1:50.000 in dilution buffer) was added to each well and incubated for 30 min at room temperature, washed three times with 200 µL washing buffer and the color reaction initiated by addition of 100 µl chromogen/substrate solution (TMB/H₂O₂) per well. Following 15 min incubation at room temperature, the stop solution was added (0.5 M sulfuric acid) and the optical density was measured at 450 nm against 620 nm as a reference. Results below 0.062 were considered negative and results above 0.063 positive.

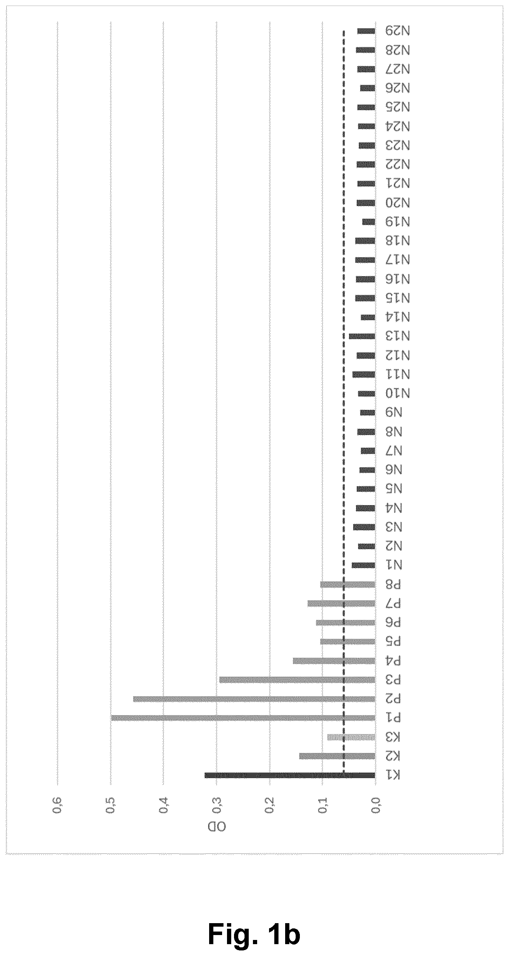

**Fig. 1b** shows the results with 29 healthy blood donors (N1-N29) and 8 samples identified as comprising autoantibodies to nephrin (P1-P8). Using the bridge capture assay, all 8 nephrin positive samples could be distinguished from the negative controls using a cutoff calculated from mean ± five times standard deviation of the signals from all healthy donors. Among the many methods used by the inventors, this is clearly and unexpectedly the superior method.

### Example 3: Detection of human antibodies to the extracellular domain of nephrin by Western blot

In order to evaluate the sensitivity of the nephrin antibody detection using standard western blotting system, 0.7 µg of the purified human recombinant extracellular domain of nephrin (SEQ ID NO: 12) per lane were separated using NuPage 4-12% bis-Tris 1.0 mm, 2D-well acrylamide gels (Thermo; NP0326BOX) with NuPage LDS Sample Buffer (4x) (Thermo; NP0007) plus 18 mM DTT and NuPage MES SDS running buffer (Thermo; NP0002). After separation, the proteins were transferred onto a nitrocellulose membrane in an NuPage transfer buffer (Thermo; NP00061). The membrane was blocked with blocking buffer (0.5 % BSA in 10 mM Tris pH 7.4, 122 mM NaCl, 0.2 % (v/v) Tween 20 ) for 15 min at room temperature, cut into stripes and placed in separated containers. Afterwards, serum samples were diluted 1:100 in blocking buffer and incubated over night at room temperature. As positive controls, anti-nephrin (SantaCruz B-12) and anti his-tag antibody (His-Tag Monoclonal Antibody; Merck chemicals GmbH 70796-3) were used. The samples were removed, and the stripes were washed three times with 2 mL washing buffer and incubated with 500 µL of alkaline phosphatase-labelled anti-human IgG conjugate or anti-mouse IgG conjugate (Dako, D0486) per stripe (available for commercial EUROIMMUN EUROLINE test kits, e.g. DL 1590-1601-1G or Dako, D0486). Following another washing step, the color reaction was initiated by addition of substrate solution, available from EUROIMMUN EUROLINE test kits, e.g. DL 1590-1601-1G, for 5 min.

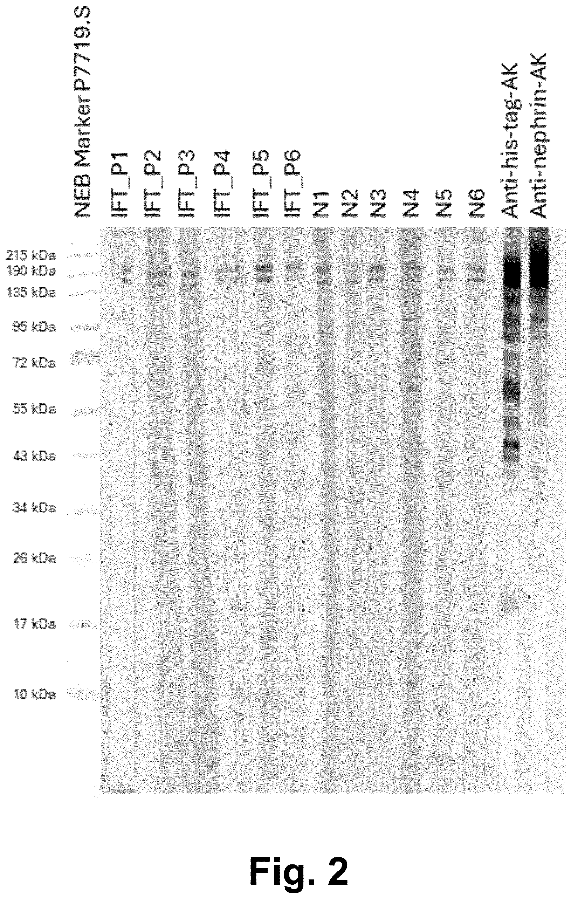

**Fig. 2** shows the western blot results using 6 serum samples, which indicated positive results in the cell based IFT described below and 6 healthy blood donors as well as anti-his-tag and anti-nephrin antibodies. In contrast to the recombinant antibodies, none of the detected sera could show a positive reaction related to anti-nephrin antibodies, showing a reduced sensitivity compared to the capture bridge assay.

### Example 4: Detection of human antibodies to the extracellular domain of human nephrin by indirect immunofluorescence

For IFT analysis, formalin fixed HEK293T cells transiently transfected with an pTriEx-1 vector expressing full-length nephrin (SEQ ID NO: 14) were used. Cells were grown in DMEM medium comprising 10% fetal calf serum (Capricorn Scientific, product number FBS-11A) and 1 X antibiotic-antimycotic (Invitrogen # 15240) at 37 °C and 5% CO₂.

For transient transfection the PEI-Max^{®} (product number 24765) was used according to the instruction of the manufacturer (Polysciences). Fixed cells were prepared by contacting cells grown on microscopy slides with formalin incubation in 1,825% (v/v) formalin in PBS for 3 minutes at room temperature. The immunoreactivity of the cells produced was confirmed using a commercial antibody to nephrin (anti-nephrin clone B-12 by Santa Cruz, product number sc-377246), which is a mouse IgG2a antibody.

The methodology and reagents for the indirect immunofluorescence were applied according to the manufacturer's instructions in the IIFT Neurology Mosaics (EUROIMMUN Medizinische Labordiagnostika AG, product number FA 112d-1). If the reaction is positive, specific antibodies of classes IgA, IgG and IgM attach to the antigens. In a second step, the attached antibodies are stained with FITC-labelled anti-human antibodies and made visible with a fluorescence microscope. Again, instructions according to the manual (EUROIMMUN Medizinische Labordiagnostika AG, product number FA112d-1) were followed.

Briefly, human serum samples were diluted (1:10) in PBS-Tween, followed by vortexing for 2 seconds. 30 µl sample per field was incubated for 30 minutes using the TITERPLANE^{™} technology, followed by washing in PBS-Tween for 1 second, followed by incubation in PBS-Tween for 5 minutes in a cuvette for thorough washing. 25 µl of human IgG specific secondary antibody conjugate was then applied and incubated for 30 minutes using the TITERPLANE^{™} technology, followed by washing in PBS-Tween for 1 second, followed by incubation in PBS-Tween for 5 minutes in a cuvette for thorough washing.

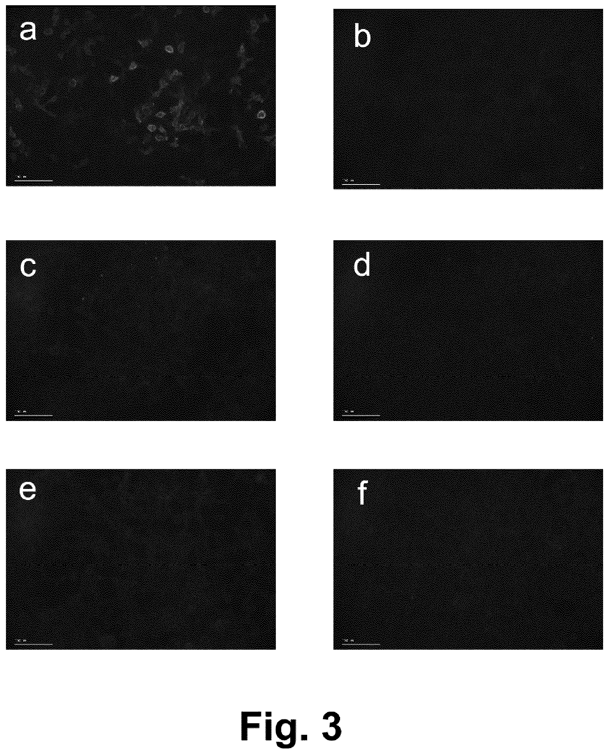

After the incubations, the carrier with the fields was covered with up to 10 µl of mounting medium per field and a glass cover slide, followed by fluorescence microscopy analysis using a EUROSTAR^{™} microscope (EUROIMMUN Medizinische Labordiagnostika AG, Lübeck). **Fig. 3** shows a representative set of data and controls.

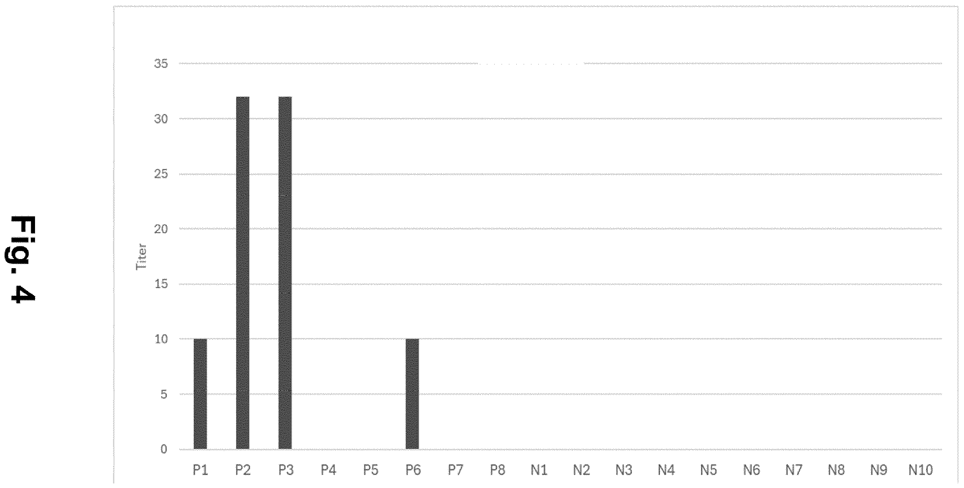

**Fig. 4** shows the results obtained with 10 healthy blood donors and 8 samples identified as comprising IgG antibodies to nephrin. Using the cell based IFT technique, only 4 of the positively characterized samples could be distinguished from the negative controls evaluated by the detected fluorescence with two (1:10 and 1:100) sample dilutions (titer). A titer of 1:32 indicated high signal visibility if the 1:10 dilution was chosen, but no visibility if the 1:100 dilution was chosen. Hence, the sensitivity of the nephrin-bridge-ELISA is superior to the detection of nephrin antibodies using indirect immunofluorescence.

### Example 5: Detection of human antibodies to the extracellular domain of human nephrin by magnetic particle based chemiluminescence assay (ChLIA)

For the detection of nephrin autoantibodies, a magnetic particle based chemiluminescence assay (ChLIA) was used. For this purpose, Dynabeads^{®} M-280 Streptavidin (Life Technologies, 11205D) magnetic beads were coated with 10 µg/mg beads of the purified human recombinant extracellular domain of nephrin carrying a C-terminal biotinylated avi tag (SEQ ID NO: 13) in phosphate buffered saline with 0,1 %(w/v) BSA, 0,2 %(v/v) Tween-20 and 0,045 %(w/v) sodium azide (coating buffer) for 10 min at room temperature. After three washing steps, the beads were incubated in coating buffer for 5 min at RT and 5 µg beads per well were placed in an LumiNunc^{™} F96 MicroWell^{™} plate. Afterwards, serum samples were diluted 1:50 in coating buffer and incubated for 15 min at room temperature. As positive control, an anti-nephrin antibody was used (K1; Clone B-12 by Santa Cruz). The samples were removed, and the wells were washed three times with coating buffer, followed by incubation with 50 µL of acridinium ester-labeled anti-human IgG conjugate per well (Anti-Human IgG-Fcy; (Jackson ImmunoResearch Europe Ltd., UK); N-Sulfpropyl-dimethyl-acridinium-N-hydroxysuccimide (Revvity, Inc., Turku, Finnland)); labeling of the antibody as described in EP 4146765 B1). After another washing step, the luminescence reaction was initiated by addition of 0,5 M sodium hydroxide and 0,2 % hydrogen peroxide solutions, the light emission was detected using a luminometer (Berthold Technologies, Centro LB 960; 38100-52) for 1 sec.

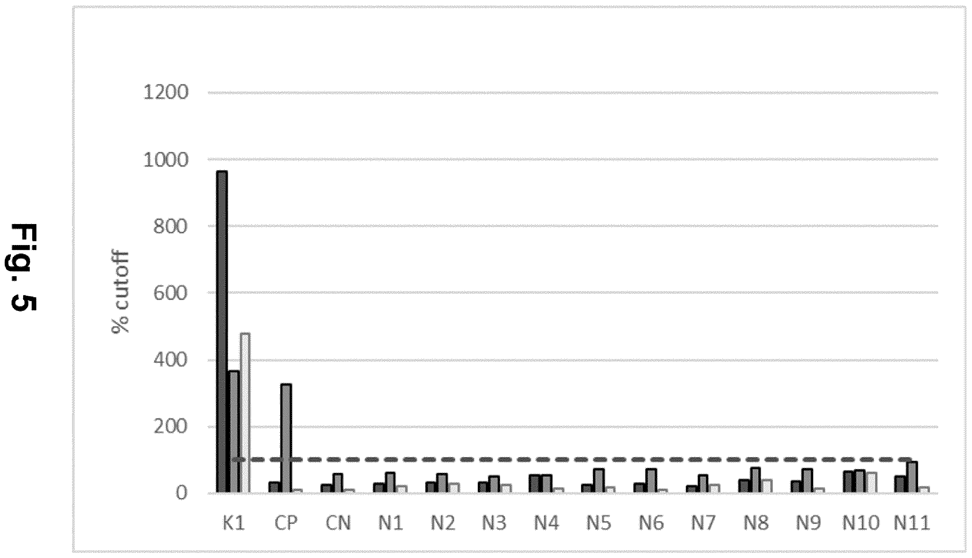

**Fig. 5** confirms the superiority of the capture bridge assay compared to conventional ELISA or ChLIA. The signals of a recombinant nephrin antibody (K1), 11 healthy blood donors (N1-N11) and two serum samples clinically characterized as positive (CP) or negative (CN) are shown in % of the cutoff signal calculated from mean ± five times standard deviation of the signals from all healthy blood donors. Only the capture bridge assay enabled a correct identification of the two patient samples CP and CN regarding nephrin autoantibody content as positive or negative, respectively.

## Claims

1. A method comprising the step detecting in a sample comprising antibodies from a mammalian subject the presence of an antibody to the extracellular domain of a mammalian nephrin, wherein the antibody is detected using a bridge capture assay.

2. A kit comprising
(a) a first polypeptide comprising an epitope from the extracellular domain of a mammalian nephrin and a second polypeptide comprising an epitope from the extracellular domain of a mammalian nephrin, wherein (i) the first polypeptide is immobilized on a solid carrier or (ii) configured for immobilization on a solid carrier (iii) or the kit comprises a solid carrier configured for immobilization of the first polypeptide and the second polypeptide is labeled or configured for labeling with a detectable label
wherein the kit comprises a solid carrier if the first polypeptide is configured for immobilization on a solid carrier,
and wherein the kit comprises a detectable label if the second polypeptide is configured for labeling with a detectable label,
or
(b) a first polypeptide comprising an epitope from the extracellular domain of a mammalian nephrin and a second polypeptide comprising an epitope from the extracellular domain of a mammalian nephrin, wherein the first polypeptide is labeled or configured for labeling with a first detectable label and the second polypeptide is labeled or configured for labeling with a second detectable label
wherein the kit comprises a first detectable label if the first polypeptide is configured for labeling with a first detectable label,
and wherein the kit comprises a second detectable label if the second polypeptide is configured for labeling with a second detectable label.

3. The kit according to claim 2, wherein the kit comprises one or more, preferably all from the group comprising a washing solution, a chemical solution reactive with the detectable label, a water-tight vessel, a positive control which is preferably an antibody to the extracellular domain of a mammalian nephrin, a calibrator, preferably a set of calibrators, a negative control, a blocking solution and a sample dilution buffer.

4. The kit according to any of claims 2 to 3, wherein the solid carrier is selected from the group consisting of a glass slide, preferably for microscopy, a biochip, a microtiter plate, a lateral flow device, a test strip, a membrane, preferably a line blot, a chromatography column and a bead, preferably a magnetic or fluorescent bead.

5. The method or kit according to any of claims 1 to 4, wherein the extracellular domain of a mammalian nephrin is from a mammalian nephrin (SEQ ID NO1), preferably selected from the group comprising human nephrin (SEQ ID NO: 2), murine nephrin (SEQ ID NO: 3), rat nephrin (SEQ ID NO: 4), rabbit nephrin (SEQ ID NO: 5), dog nephrin (SEQ ID NO: 6), cat nephrin (SEQ ID NO: 7), horse nephrin (SEQ ID NO: 8), sheep nephrin (SEQ ID NO: 9), goat nephrin (SEQ ID NO: 10) and camel nephrin (SEQ ID NO: 11), more preferably human nephrin (SEQ ID NO: 2).

6. The method or kit according to any of claims 1 to 5, wherein the presence of the antibody is detected using a detection method selected from the group consisting of immunodiffusion, light scattering immunoassays, labeled immunoassays such as those from the group comprising radiolabeled immunoassays, enzyme immunoassays, chemiluminescence immunoassays, preferably electrochemiluminescence immunoassays, and fluorescence immunoassays.

7. The method or kit according to any of claims 1 to 6, wherein the method or kit is for aiding in diagnosing whether a subject suffers from of a nephrological autoimmune disease associated with the presence of an antibody to nephrin or for determining whether a sample comprising antibodies is from a subject more likely to suffer from of a nephrological autoimmune disease associated with the presence of an antibody to nephrin than a healthy reference subject.

8. The method or kit according to any of claims 1 to 6, wherein the method or kit is for monitoring the progression in a subject of a nephrological autoimmune disease associated with the presence of an antibody to nephrin.

9. The method or kit according to any of claims 1 to 6, wherein the method or kit is for evaluating in a subject suffering from a nephrological autoimmune disease associated with the presence of an antibody to nephrin the response to an immunosuppressive treatment of said disease.

10. The method or kit according to any of claims 7 to 9, wherein the autoimmune disease is selected from the group comprising minimal change disease, FSGS and idiopathic nephrotic syndrome.

11. The method or kit according to any of claims 1 to 6, wherein the method or kit is for evaluating the chances of a successful kidney transplantation or monitoring after a kidney transplantation.

12. The method or kit according to any of claims 1 to 6, wherein the method or the kit is for selecting patients suffering from an autoimmune disease, preferably a nephrological autoimmune disease to be included in a cohort for a clinical study aiming to test the efficacy of immunosuppressive drugs, preferably systemic immunomodulators.

13. The method according to any of claims 1 and 5 to 12, wherein the subject is selected from the group comprising a primate, preferably a human, a mouse, a rat, a dog, a rabbit, a non-human primate, a goat, a guinea pig, a pig, a sheep, a camel, a horse a hamster, an alpaca, a llama and a cow.

14. The method or kit according to any of claims 1 to 13, wherein method is or the kit is configured for the detection of the antibody in a sample which is selected from the group comprising whole blood, capillary blood, serum, plasma, and a dried blood spot, which is preferably used undiluted.

15. The method or kit according to any of claims 1 to 14, wherein the detection of the method is a qualitative detection, preferably a semi-quantitative detection, more preferably a quantitative detection or the kit is configured for a qualitative detection, preferably a semi-quantitative detection, more preferably a quantitative detection of the antibody.
